(19) Europäisches Patentamt
European Patent Office
Office européen des brevets

(11) Publication number: 0 000 034
B1

(12) **EUROPEAN PATENT SPECIFICATION**

(45) Date of publication of patent specification: **23.05.84**

(21) Application number: **78100057.5**

(22) Date of filing: **01.06.78**

(51) Int. Cl.³: **C 07 C 91/32,** C 07 C 93/26, A 61 K 31/135 // C07C93/14, C07C131/00, C07C49/255

(54) **Fluorinated amines, compositions and process for preparing said compounds.**

(30) Priority: **01.06.77 US 802388**

(43) Date of publication of application: **20.12.78 Bulletin 78/01**

(45) Publication of the grant of the patent: **23.05.84 Bulletin 84/21**

(84) Designated Contracting States: **BE CH DE FR GB LU NL SE**

(56) References cited:
**GB-A-1 218 135**
**US-A-3 046 300**

(73) Proprietor: **MERCK & CO. INC.**
**126, East Lincoln Avenue P.O. Box 2000**
**Rahway New Jersey 07065 (US)**

(72) Inventor: **Kollonitsch, Janos**
**3 Plymouth Road**
**Westfield, N.J. (US)**
Inventor: **Marburg, Stephen**
**50 Concord Avenue**
**Metuchen, N.J. (US)**
Inventor: **Patchett, Arthur Allan**
**207 Oak Lane**
**Cranford, N.J. (US)**

(74) Representative: **Abitz, Walter, Dr.-Ing. et al**
**Abitz, Morf, Gritschneder P.O. Box 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 000 034 B1

## Description

Background of the invetion

The present invention is concerned with certain difluoro- or trifluoromethyl phenylethylamines.

α-Methyl-3,4-dihydroxyphenylalanine, particularly its L-isomer, is a known antihypertensive agent. (U.S. 2,868,818; U.S. 3,344,023).

α-Trifluoromethyloxyphenylalanines are known compounds which are specific in their physiological activity in vivo (U.S. 3,046,300). They reduce the norepinephrine to be found in the heart but have no effect on that to be found in the brain. Further, while they appear in vitro to be active as inhibitors of mammalian decarboxylase, they do not appear to have this activity in vivo. They can also be used as intermediates in the preparation of norepinephrine-like compounds by decarboxylation of the amino acid followed by hydroxylation of the β-carbon of the resulting amine.

Novel α-difluoromethyl- or α-trifluoromethyl-3,4-di-OR-ethylamines have been discovered. These compounds have biological activity including decarboxylase inhibition.

Summary of the invention

α-difluoromethyl- or α-trifluoromethyl-3,4-OR-phenylethylamines and their salts.

Description of the invention

The present invention is embodied in compounds having the formula

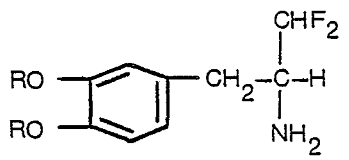

I

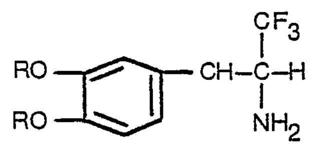

II

wherein

R is H or $C_2$—$C_6$ alkanoyl.

The pharmaceutically acceptable salts of the formula I—II compounds are also included. These salts generally are acid addition salts of suitable organic or inorganic acids. Preferred salts are the hydrohalides such as the hydrobromides, the hydrochlorides, the hydrogen iodides. Most preferred salts are the hydrochlorides.

The compounds of formula I—II have a chiral center and may occur in optically active forms, i.e., as optical isomers. These isomers are conventionally designated as D and L, d and 1, + and —, (S) and (R) or by a combination of these symbols. Where the compound name or formula does not specify the isomer form, all forms are included, i.e., the individual isomers, mixtures thereof and racemates.

Examples of suitable alkanoyl groups for R are acetyl, pivaloyl, 2-methylpropanoyl, butanoyl and the like. The most preferred R substituent is hydrogen.

A preferred compound is the trifluoromethyl compound having the formula

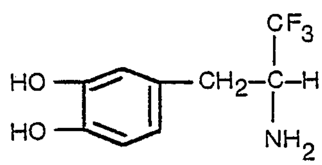

III

The R-isomer of III is more preferred.

Another preferred compound of the present invention is that having the formula

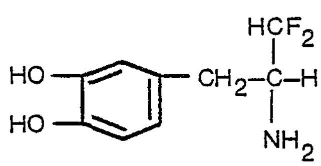

IV

The R-isomer form of the formula IV compound is also more preferred.

The compounds of the present invention have 3,4-dihydroxyphenylalanine (DOPA) decarboxylase

inhibiting activity. This activity was determined by conventional in-vitro assay procedures. This biological activity permits the present compounds to be used as diagnostic tools to determine the presence and importance of the corresponding decarboxylase in relation to diseases or to functioning of biological systems. Also the present, compounds may be used in combination with DOPA to improve DOPA's effectiveness in treating Parkinson's disease.

In addition, some of these compounds also have pharmaceutical activity as antihypertensive agents. Thus, these compounds are useful for treating hypertension in humans.

For treating hypertension, the compounds or their salts may be administered to the hypertensive patient orally, parenterally or via any other suitable administration route. Conventional dosage forms are used such as tablets, troches, capsules, liquid formulations, e.g. solutions, dispersions, emulsions, elixirs and the like. Conventional compounding ingredients, i.e., diluents, carriers, etc., and conventional preparation procedures are utilized.

Compounds of the present invention may be prepared by any convenient process.

One such process scheme for preparing the difluoro compounds is illustrated by the following reaction sequence:

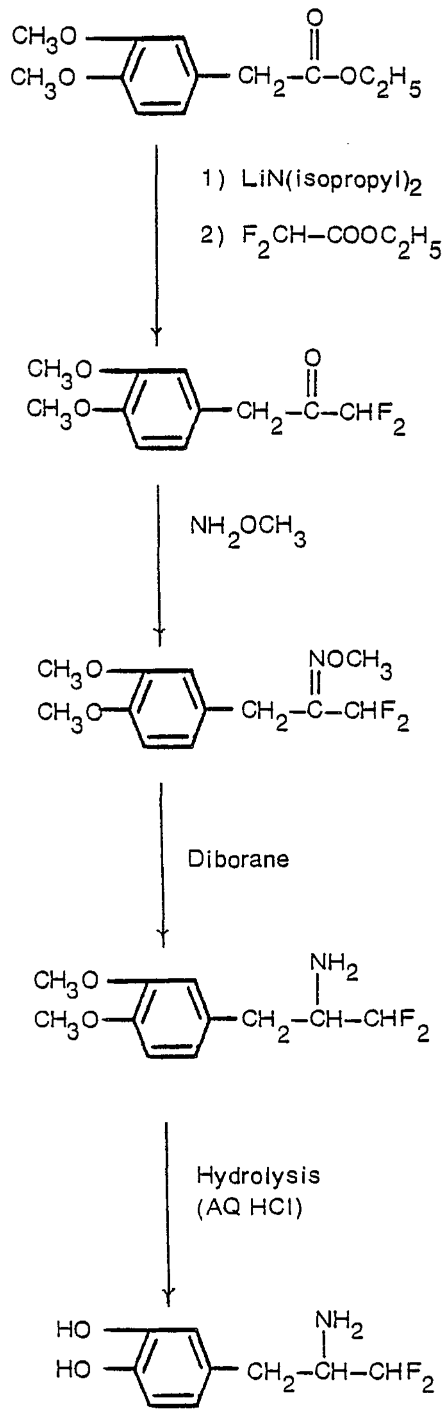


I

2

3

Homoveratric acid and its derivatives such as dialkyl amides, esters and thioesters may be transformed into an anionic species with such bases as metalated dialkyl amides (e.g., lithium diisopropylamide), metalated alkyls (e.g., butyl lithium), hydrides (NaH) or metal amides ($KNH_2$). Such anions may be condensed with activated forms of difluoroacetic acid such as its ester, or acid chlorides. Except in the case of homoveratric acid itself, these condensation products require hydrolysis to effect decarboxylation to the difluoromethyl veratryl ketone *1*. A carbon nitrogen bond may be introduced at the ketonic position with the usual amine reagents such as $NH_3$ and hydroxylamine. However, this is most conveniently done by using methoxy amine to form the ketone O-methyl oxime, *2*. This compound may be reduced to the amine *3* using metal catalysts and hydrogen, active metals (e.g., Zn) and most conveniently boranes such as $BH_3$. The cleavage of the phenol ethers can be accomplished with reagents such as $BBr_3$ but is easily effected by heating with hydrohalic acids such as aq. HCl, aq. HBr and the like.

A process for preparing the trifluoro compounds is illustrated by the following reaction scheme:

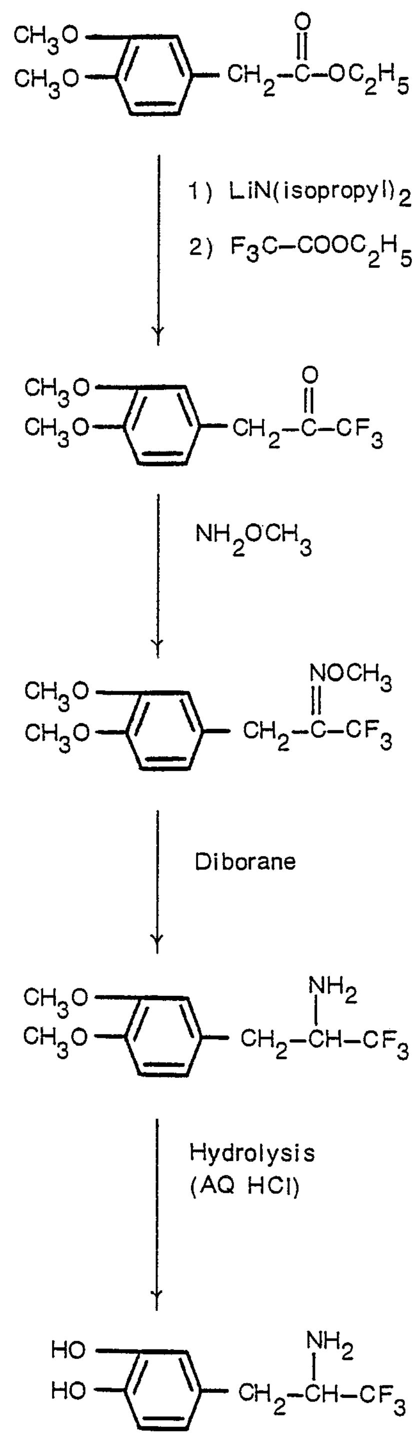


5

6

7

8

Homoveratric acid and its derivatives such as dialkyl amides, esters and thio esters may be transformed into an anionic species with such bases as metalated dialkyl amides (e.g., lithium diisopropylamide), metalated alkyls (e.g., butyl lithium), hydrides (NaH) or metal amides ($KNH_2$). Such anions may be condensed with activated forms of trifluoroacetic acid such as its ester or acid chloride. Except in the case of homoveratric acid itself, these condensation products require hydrolysis to effect decarboxylation to the trifluoromethyl veratryl ketone *5*. A carbon nitrogen bond may be introduced at the ketonic position with the usual amine reagents such as ammonia and hydroxylamine. However, this is most conveniently done by using methoxy amine ($NH_2OCH_3$) to form the ketone O-methyl oxime *6*. This compound may be reduced to the amine 7 using metal catalysts and hydrogen, active metals and most conveniently boranes such as $BH_3$. The cleavage of the phenol ethers can be accomplished with reagents such as $BBr_3$ but is easily effected by heating with hydrohalic acids such as aqueous HCl, aqueous HBr and the like.

The pharmaceutically acceptable salts of the present compounds may be obtained directly from the acid hydrolysis steps described above. Such salts may also be obtained by treatment of the formula I—II free base with an appropriate acid under suitable conditions.

Where the compounds of the present invention are obtained as racemates, they may be separated into the individual enantiomers by conventional resolution techniques. Such techniques commonly involve the formation of a salt of a present racemic amine with an optically active acid.

Compounds of the present invention where R is lower alkanoyl are prepared by appropriately acylating the corresponding compound where R is H, as illustrated by the following equation:

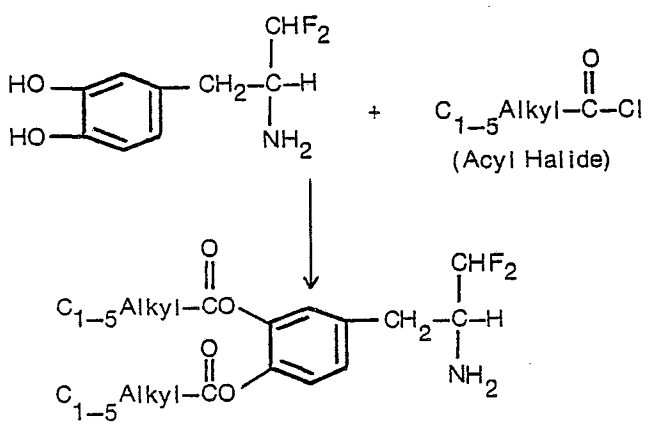


To prevent acylation of the $\alpha$-$NH_2$ group the reaction may be carried out in an acid medium, e.g., glacial acetic acid. An example illustrating such an acylation system is in U.S. 3,983,138.

THe following examples illustrate the preparation of compounds of the present invention. All temperatures are in °C. The melting points were obtained in open capillary and are uncorrected.

Example 1

*Preparation of $\alpha$-difluoromethyl-2-(3',4'-dihydroxyphenyl)ethylamine*

a. Veratryl difluoromethyl ketone (1)

A 1-liter three-neck flask, fitted with a magnetic stirrer, condenser, nitrogen inlet, and septum was charged with 23 ml of diisopropylamine (176 mmoles) and 50 ml of tetrahydrofuran (THF). The solution was cooled to 0°C followed by addition of 76 ml of 2.1M n-butyl lithium in hexane (159.6 mmoles) over a period of 15 min. The solution was then cooled to —78°C and to it was added 15.68 g of homoveratric acid (80 mmoles) in 80 ml of THF. The addition required 25 min and the temperature was not permitted to exceed —40°C. On completion of the addition, 35 ml of THF were added and the resulting mixture was stirred in an ice bath for 3 hours. It was then recooled to —78°C and 10.3 g of ethyl difluoroacetate (83 mmoles) in 90 ml of THF were added. The mixture was then aged at —78°C for 2 hrs, and 1 hr. at 0°C, after which it was worked up by quenching onto 600 ml of 2N HCl, extracting with 3 × 300 ml of ethyl acetate, backwashing with 5% aq. $NaHCO_3$ and saturated NaCl solution. The organic solution was concentrated to give veratryl difluoromethyl ketone (*1*); this compound forms a crystalline 2,4-dinitrophenyl hydrazone, m.p. 107—109°C. Small amounts of this ketone were purified by preparative thin layer chromatography as follows: A 1000$\mu$ silica gel plate was spotted with 175 mg of crude *1* and developed with a 70:30 chloroform:acetone mixture affording about 110 mg of single spot material.

b.) Veratryl difluoromethyl ketone O-methyl oxime (2)

125 mg of *1* was dissolved in 1.1 ml of pyridine and treated with 125 mg of methoxyamine hydrochloride and the mixture was allowed to remain overnight at room temperature. It was then quenched on water. The aqueous mixture was extracted with ethyl acetate, dried and concentrated to

120 mg of single spot material (70:30 chloroform:acetone, silica plates). This material had an NMR spectrum which was consistent with a syn-anti mixture of the desired oxime *2.*

c.) R,S-1-Difluoromethyl-2-(3',4'-dimethoxyphenyl)ethylamine hydrochloride (*3*)

120 mg of *2* was charged to a 2-neck 15-ml flask fitted with a septum and condenser and $N_2$ inlet. The material was dissolved in 1 ml of THF, cooled in an ice bath. After 1.5 ml 1M $BH_3$ in THF had been slowly added through the septum using a syringe, the solution was stirred at reflux for 2 hrs. and then at room temperature for 16 hrs. It was then quenched onto 10 ml of methanol. To this was added 1 ml conc. HCl and the solution aged for 2 hrs. and then concentrated to dryness to afford R,S-1-difluoromethyl-2-(3',4'-dimethoxyphenyl)ethylamine hydrochloride (*3*), m.p. 164—167°C (mass spectrum m/e =231).

d.) R,S-1-Difluoromethyl-2-(3',4'-dihydroxyphenyl)ethylamine heminaphthalene 1,5-disulfonate (*4*)

650 mg of *3* was dissolved in 13 ml of conc. HCl and the solution heated under $N_2$ in a sealed tube at 130°C for 30 min. The solution was concentrated to dryness *in vacuo,* the residue applied to a 15-ml AG—50X8 (200—400 mesh) Dowex* 50 cation echange resin column H+ form. The column was washed with 250 ml $H_2O$ and then eluted with 3N HCl containing 10% methanol. 20 ml fractions were collected and the effluent monitored by a UV detector. Fractions 2—51 were combined and concentrated to give *R*,S-1-difluoromethyl-2-(3',4'-dihydroxyphenyl)ethylamine hydrochloride. 360 mg of this material were dissolved in 3 ml of 2-propanol and 216 mg of naphthalene 1,5-disulfonic acid were added. The solution was concentrated to dryness and the residue redissolved in 2-propanol and reconcentrated. This procedure was repeated three times (to remove HCl). Before the final concentration, the solution was filtered through analytical filter aid (Celite*). Concentration followed by trituration with acetonitrile afforded the hemi naphthalene 1,5-disulfonate *4*, m.p. 244—247°C Dec.

Example 2

*Preparation of R,S-1-Trifluoromethyl-2-(3',4'-dihydroxyphenyl)ethylamine*

a. Veratryl trifluoromethyl ketone *(5)*

A 250 ml. flask fitted with stirrer, condenser, nitrogen inlet and septum was charged with 2.76 ml of diisopropylamine and 20 ml of THF. The solution was cooled to 0°C. followed by addition of 19 ml of 2.1M butyl lithium in hexane over a period of 15 minutes. The solution was then cooled to —78°C and to it was added 3.92 g of homoveratric acid in 50 ml of THF. The flask was removed from the —78° bath and immersed in an ice bath for 3 hours. The solution was recooled to —78°C. and to it was added 2.84 g ethyl trifluoroacetate in 20 ml of THF. After stirring at —78° for 0.5 hours, the solution was warmed to 0° and stirred for 1.5 hours at this temperature.

It was then quenched into 100 ml 3N HCl, which was then extracted with 3 x 75 ml of ethyl acetate, backwashed with $H_2O$ and 5% aqueous $NaHCO_3$ and saturated NaCl solution. The organic layer was dried over $Na_2SO_4$ and concentrated to give crude veratryl trifluoromethyl ketone (*5*). This was purified by thin layer chromatography (80:20 chloroform:acetone, silica gel) and recrystallized from cyclohexane to afford pure *5*, m.p. 86—88°C.

b.) Veratryl trifluoromethyl ketone-O-methyl oxime (*6*)

530 Mg of *5* was dissolved in 3 ml of pyridine and treated with 500 mg of methoxyamine hydrochloride and the resulting solution was allowed to remain at room temperature overnight. The pyridine was concentrated to a small volume and the residue extracted with ethyl acetate and water. Drying and concentration of the organic layer afforded the crude O-methyl oxime *6*. Purification was effected by thin layer chromatography (80:20 chloroform:acetone, silic gel) and the lead spot proved to be pure *6*.

c.) R,S-1-trifluoromethyl-2-(3',4'-dimethoxyphenyl)ethylamine (*7*)

200 Mg of *6* was dissolved in 2 ml of THF and charged to a 15 ml 2-neck flask fitted with a septum and condenser. The flask was cooled in an ice bath and 3 ml of 1M $BH_3$ in THF was added. The solution was then refluxed for 1.5 hours and then allowed to remain at room temperature overnight. The solution was quenched into 5 ml of methanol and 1 ml of concentrated HCl was added. After aging for 2 hours, it was concentrated, redissolved in 5 ml of concentrated HCl and reconcentrated to 250 mg of crude *7* hydrochloride. The material was dissolved in $H_2O$, basified with 2.5N NaOH and extracted with ethyl acetate. The organic layer was dried and concentrated to give R,S-1-trifluoromethyl-2-(3',4'-dimethoxyphenyl)ethylamine (*7*).

(The hemi naphthalene 1,5-disulfonate salt was prepared by treating 40 mg of *7* with 23 mg of naphthalene 1,5-disulfonic acid in 2-propanol and concentrating to dryness. The residue was twice redissolved and reconcentrated and then triturated with acetonitrile to give the crystalline hemi naphthalene disulfonate salt of *7*, m.p. 265—268°C.).

---

*Registered Trade Mark, at least in the United Kingdom.

d.) R,S-1-trifluoromethyl-2-(3′,4′-dihydroxyphenyl)ethylamine (*8*)

730 Mg of *7* was dissolved in 7 ml concentrated HCl and heated at 130° in a sealed tube for 1.25 hours. The solution was then concentrated to 725 mg of crude hydrochloride of *8*. This was dissolved in 20 ml of 2-propanol and treated with 406 mg of naphthalene 1,5-disulfonic acid. The solution was concentrated to dryness and redissolved in 2-propanol. This procedure was repeated twice and finally concentrated to dryness. Trituration of the residue with 30 ml of acetonitrile afforded the hemi naphthalene 1,5-disulfonate of R,S-1-trifluoromethyl-2-(3′,4′-dihydroxyphenyl)ethylamine (*8*), m.p. 287—290°C. dec.

The R,S-1-trifluoromethyl-2-(3′,4′-dihydroxyphenyl)ethylamine is obtained by conventional neutralization of the disulfonate.

**Claims**

1. Compounds having the formula

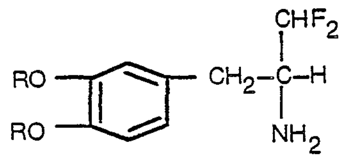

I

wherein R is H or $C_2$—$C_6$ alkanoyl and pharmaceutically acceptable salts thereof.

2. Compound of Claim 1 wherein R is H, having the R-isomer configuration.

3. Compounds having the formula

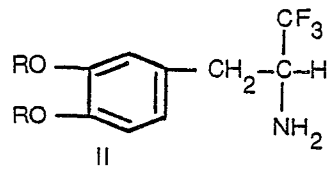

II

wherein R is H or $C_2$—$C_6$ alkanoyl, and pharmaceutically acceptable salts thereof.

4. Compound of Claim 3 having the formula

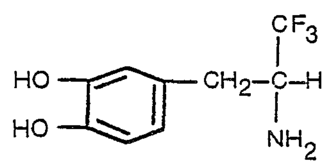

having the R-isomer configuration.

5. A pharmaceutical composition containing a compound of Claims 1 to 4.

6. A process for preparing a compound of Claim 1 wherein R is hydrogen which comprises hydrolyzing a compound having the formula

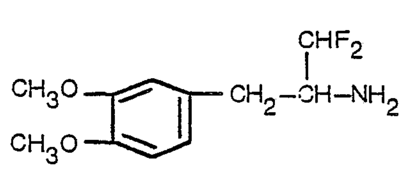

to obtain said Claim 1 compound (I) where R is H

7. A process for preparing a compound of Claim 3 wherein R is hydrogen which comprises hydrolyzing a compound of the formula

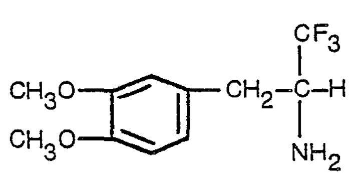

to obtain said Claim 3 compound (II), where R is H.

**Revendications**

1. Composés ayant pour formule

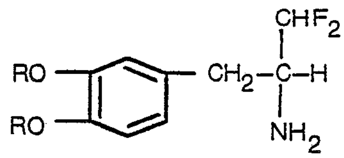

I

dans laquelle R est H ou un alcanoyle en $C_2$—$C_6$ et leurs sels acceptables en pharmacie.

2. Composé de la revendication 1, dans lequel R est H, ayant la configuration de l'isomère R.

3. Composés ayant pour formule

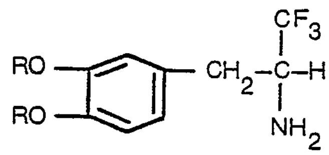

II

dans laquelle R est H ou un alcanoyle en $C_2$—$C_6$ et leurs sels acceptables en pharmacie.

4. Composé de la revendication 3 ayant pour formule

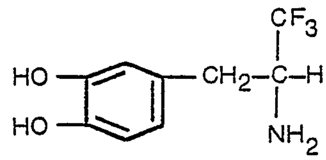

ayant la configuration de l'isomère R.

5. Une composition pharmaceutique contenant un composé des revendications 1 à 4.

6. Un procédé pour préparer un composé de la revendication 1 dans lequel R est un hydrogène, qui comprend l'hydrolyse d'un composé ayant pour formule

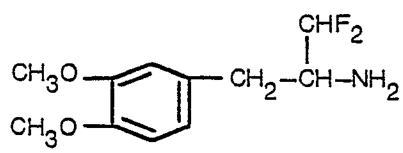

pour obtenir ledit composé (I) de la revendication 1 dans lequel R est H.

7. Un procédé pour préparer un composé de la revendication 3 dans lequel R est un hydrogène, qui comprend l'hydrolyse d'un composé de formule

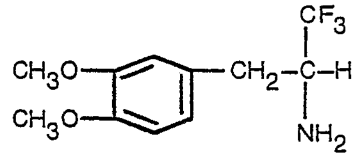

pour obtenir ledit composé (II) de la revendication 3 dans lequel R est H.

**Patentansprüche**

1. Verbindungen mit der Formel

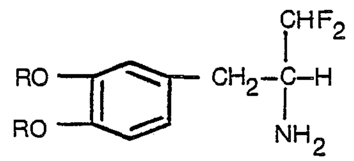

I

worin R H oder $C_2$—$C_6$-Alkanoyl ist und pharmazeutisch brauchbare Salze davon.

2. Verbindung nach Anspruch 1, worin R H ist, mit der Konfiguration des R-Isomeren.

3. Verbindungen mit der Formel

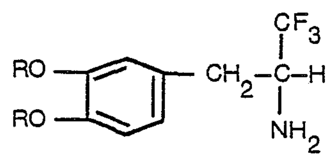

II

worin R H oder $C_2$—$C_6$-Alkanoyl ist und pharmazeutisch brauchbare Salze davon.

4. Verbindung nach Anspruch 3 mit der Formel

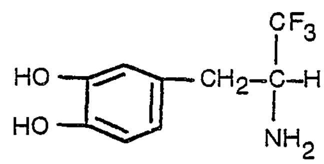

mit der Konfiguration des R-Isomeren.

5. Pharmazeutische Zusammensetzung, enthaltend eine Verbindung der Ansprüche 1 bis 4.

6. Verfahren zur Herstellung einer Verbindung nach Anspruch 1, worin R Wasserstoff ist, durch Hydrolysieren einer Verbindung mit der Formel

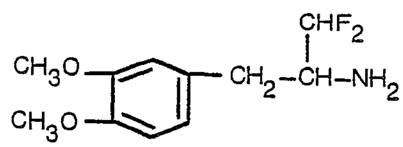

unter Erzielung der Verbindung I, nach Anspruch 1, worin R H ist.

7. Verfahren zur Herstellung einer Verbindung nach Anspruch 3, worin R Wasserstoff ist, durch Hydrolysieren einer Verbindung der Formel

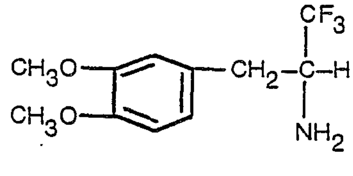

unter Erzielung der Verbindung II, nach Anspruch 3, worin R H ist.